# EUROPÄISCHE PATENTSCHRIFT

(11) **EP 3 218 334 B1**
(45) Veröffentlichungstag und Bekanntmachung des Hinweises auf die Patenterteilung: **26.09.2018**
(21) Anmeldenummer: 15790976.3
(22) Anmeldetag: 09.11.2015
(51) Int. Cl.: C07C 4/06, C07C 5/48, C07C 7/08, C07C 7/09, C07C 7/11, C07C 7/148, C07C 11/167, C07C 11/08

(54) **VERFAHREN ZUR HERSTELLUNG VON 1,3-BUTADIEN DURCH DEHYDRIERUNG VON N-BUTENEN UNTER BEREITSTELLUNG EINES BUTANE UND 2-BUTENE ENTHALTENDEN STOFFSTROMES**
METHOD FOR PREPARING 1,3-BUTADIENE BY DEHYDROGENATION OF N-BUTENES PROVIDING A BUTANE AND MIXTURES OF MATERIALS CONTAINING 2-BUTENE
PROCÉDÉ DE FABRICATION DE 1,3 BUTADIÈNE PAR LA DÉSHYDRATATION DE N-BUTÈNES PAR PRÉPARATION D'UN FLUX DE MATIÈRE CONTENANT DU BUTANE ET DE 2-BUTÈNE

(30) Priorität: 14.11.2014 EP 14193247
(43) Veröffentlichungstag der Anmeldung: 20.09.2017
(73) Patentinhaber: BASF SE, 67056 Ludwigshafen (DE); Linde Aktiengesellschaft, 80331 München (DE)
(72) Erfinder: UNGELENK, Jan, 67141 Neuhofen (DE); GRÜNE, Philipp, 68161 Mannheim (DE); WALSDORFF, Christian, 67061 Ludwigshafen (DE); JOSCH, Jan Pablo, 67434 Neustadt (DE); BENDER, Michael, 67063 Ludwigshafen (DE)
(74) Vertreter: Schuck, Alexander
(86) Internationale Anmeldenummer: PCT/EP2015/076018
(87) Internationale Veröffentlichungsnummer: WO 2016/075065

(56) Entgegenhaltungen:
- WO-A1-2014/160825
- US-A- 3 914 332
- US-A1- 2013 281 748

## Beschreibung

Die Erfindung betrifft ein Verfahren zur Herstellung von 1,3-Butadien durch Dehydrierung von n-Butenen aus der C4-Produktfraktion einer Fluid-Catalytic-Cracking (FCC)-Anlage unter Bereitstellung eines Butane und 2-Butene enthaltenden Stoffstromes.

Butadien ist eine bedeutende Grundchemikalie und wird beispielsweise zur Herstellung von Synthesekautschuken (Butadien-Homopolymere, Styrol-Butadien-Kautschuk oder NitrilKautschuk) oder zur Herstellung von thermoplastischen Terpolymeren (Acrylnitril-Butadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Ethylbenzol oder Styrol dehydriert werden kann.

1,3-Butadien kann durch thermische Spaltung gesättigter Kohlenwasserstoffe hergestellt werden. Ein übliches Verfahren ist das Steam-Cracken von Naphtha, wobei ein KohlenwasserstoffGemisch aus Methan, Ethan, Ethen, Acetylen, Propan, Propen, Propin, Allen, Butanen, Butenen, Butadienen , Butinen, Methylallen, C₅- und höheren Kohlenwasserstoffen an. Der Anteil an Butadien an der Produktion eines Steam-Crackers ist abhängig von den Betriebsbedingungen ("Schärfe") der Crack-Stufe, vor allem aber auch von der Zusammensetzung des Feed-Stroms. So ist die Ausbeute an 1,3-Butadien bei Verwendung von Naphtha als Feed tendenziell höher als bei Verwendung von leichtsiedenden Kohlenwasserstoffen, während bei Verwendung von Ethan als Feed praktisch keine wirtschaftlich relevanten Mengen an Butadien im Crack-Prozess erhalten werden. Durch eine Änderung der Rohstoffversorgung von Steam-Crackern oder eine stärker steigende Nachfrage nach Butadien kann deshalb eine lokale, regionale oder globale Unterversorgung mit Butadien entstehen, wobei der Bedarf durch Butadien aus Steam-Crackern allein nicht zu decken ist.

Zunehmende Bedeutung erlangen daher Verfahren zur on-purpose Herstellung von Butadien, z.B. durch Dehydrierung von n-Butenen, sowie die Kombination solcher Verfahren mit klassischen Crack-Verfahren zur Maximierung der Gesamtausbeute an Butadien.

So wird beispielsweise ein Verfahren zur Herstellung von Butadien durch Dehydrierung von n-Butenen in WO 2013/106039 beschrieben. Ein Nachteil solcher nicht-oxidativen Verfahren sind in der Regel die durch die Lage des thermodynamischen Gleichgewichts stark limitierten Umsätze. Oftmals sind auch große Mengen Dampf zur Verschiebung des Gleichgewichts oder zur Erhaltung der Katalysatorstabilität erforderlich.

1,3-Butadien kann auch durch oxidative Dehydrierung von n-Butenen (1-Buten und/oder 2-Butenen) erhalten werden. Verfahren zur oxidativen Dehydrierung von Butenen zu Butadien sind grundsätzlich bekannt.

US 2012/0130137 A1 beispielsweise beschreibt ein solches Verfahren unter Verwendung von Katalysatoren, die Oxide von Molybdän, Bismuth und in der Regel weiteren Metallen enthalten. Für die nachhaltige Aktivität solcher Katalysatoren für die oxidative Dehydrierung ist ein kritisches Mindestmaß an Sauerstoffpartialdruck in der Gasatmosphäre erforderlich, um eine zu weitgehende Reduktion und damit einen Performanceverlust der Katalysatoren zu vermeiden. Aus diesem Grund kann in der Regel auch nicht mit einem stöchiometrischen Sauerstoffeinsatz oder vollständigem Sauerstoff-Umsatz im Oxidehydrierungsreaktor (ODH-Reaktor) gearbeitet werden. In US 2012/0130137 A1 ist zum Beispiel ein Sauerstoffgehalt von 2,5 bis 8 Vol.-% im Ausgangsgas beschrieben.

Eine wesentliche Hürde für die wirtschaftliche Einführung von Verfahren zur oxidativen Dehydrierung von n-Butenen zu 1,3-Butadien ist der verfahrenstechnische Aufwand, der zu hohen spezifischen Investitions- und Betriebskosten für das so hergestellte 1,3-Butadien führt. 1,3-Butadien lässt sich mittels Extraktion aus dem C4-Schnitt von Steam-Crackern erheblich günstiger herstellen.

WO 2013/098760 beschreibt ein Verfahren zur Erhöhung der extrahierbaren Menge an Butadien aus einem C4-Raffinat, wie es in einem Steam-Cracker anfällt, mit den Schritten:
a) Abtrennung von 1,3-Butadien aus einer Mischung enthaltend Butane, Butene und 1,3-Butadien mittels einer Extraktivdestillation in einer Extraktionseinheit, wobei ein sogenanntes Raffinatprodukt enthaltend Butane und Butene erhalten wird;
b) Dehydrieren des Raffinatprodukts in der Dehydriereinheit in Gegenwart eines Dehydrier-Katalysators und eines inerten Verdünnungsgases, so dass ein 1,3-Butadien enthaltender Austragsstrom erhalten wird;
c) Rückführung des 1,3-Butadien enthaltenden Austragsstroms nach Abtrennung der nicht kondensierbaren Bestandteile direkt in die Extraktionseinheit.

Nachteilig am in der WO2013/098760 beschriebenen Verfahren ist insbesondere, dass die Qualität des die Extraktionsstufe verlassenden Raffinatprodukts durch die Rückführung des 1,3-Butadien enthaltenden Austragsstroms aus der Dehydrierstufe in die Extraktionsstufe verändert wird, dahingehend dass der Anteil an solchen Bestandteilen erhöht wird, welche sich in der Dehydrierstufe weniger gut zu 1,3-Butadien umsetzen. Bei vielen oxidativen Dehydrierverfahren in Gegenwart von Sauerstoff (ODH) werden im Wesentlichen nur lineare Butene zu 1,3-Butadien umgesetzt, während sich n- und iso-Butan weitgehend inert verhalten und iso-Buten sehr unselektiv mit negativen Auswirkungen auf die Katalysatorperformance umgesetzt wird, und die aus iso-Buten entstehenden Nebenprodukte, z.B. Methacrolein, in den Aufarbeitungsschritten stören können.

WO 2014/160825 A1 beschreibt ein zum Verfahren in WO 2013/098760 sehr ähnliches Verfahren zur Erhöhung der extrahierbaren Menge an Butadien aus einem C4-Raffinat, wie es in einem Steam-Cracker anfällt, mit den Schritten:
a) Abtrennung von 1,3-Butadien aus einer Mischung enthaltend Butane, Butene und 1,3-Butadien mittels einer Extraktivdestillation in einer Extraktionseinheit, wobei ein erster C4-Strom enthaltend Butane und Butene erhalten wird (Raffinatprodukt);
b) Abtrennung von iso-Buten aus dem ersten C4-Strom, wobei einer zweiter C4-Strom erhalten wird;
c) Dehydrieren des zweiten C4-Stroms in der Dehydriereinheit, so dass ein 1,3-Butadien enthaltender Austragsstrom erhalten wird;
d) Einleitung des 1,3-Butadien enthaltenden Austragsstroms in eine Extraktionseinheit bzw. Rückführung des 1,3-Butadien enthaltenden Austragsstroms in die unter a) genannte Extraktionseinheit

Abtrennung von iso-Buten bedeutet hier, dass der überwiegende Teil von iso-Buten aus dem Einsatzgasstrom a abgetrennt wird. Typischerweise werden mehr als 60 % der iso-Butene abgetrennt, bevorzugt mehr als 80 %, besonders bevorzugt mehr als 90 % und ganz besonders bevorzugt mehr als 95 %. Das Verhältnis von iso-Buten zu n-Butenen im Stoffstrom b beträgt dann typischerweise weniger als 0,2, bevorzugt weniger als 0,1 und ganz besonders bevorzugt weniger als 0,05.

Das in der WO 2014/160825 A1 beschriebene Verfahren unterscheidet sich gegenüber dem in der WO2013/098760 beschriebenen Verfahren im Wesentlichen dadurch, dass der Ausgangsstrom der Dehydriereinheit vor der Rückführung in die Extraktionseinheit in mehreren Stufen aufwändig aufbereitet wird. Insbesondere wird Butan, welches sich in der Dehydriereinheit weitgehend inert verhält, vor der Rückführung abgetrennt. Die Aufreinigung ist mit hohen Kosten für Energie oder Investment verbunden.

KR 20130046259 A1 beschreibt einen Prozess zur Verwendung eines Raffinat-3 genannten Stroms aus einer Olefin-Metathese als Eingangsstrom zur Herstellung von 1,3-Butadien durch oxidative Dehydrierung von n-Butenen. In einer Ausführungsform wird eine in einem Naphtha-Cracker (Steam-Cracker mit Naphtha-Feed) erhaltene Roh-C₄-Fraktion zunächst in eine Extraktionsstufe gefahren. Dabei wird Butadien abgetrennt und ein sogenanntes Raffinat-1 erhalten. In einer weiteren Verfahrensstufe wird aus dem Raffinat-1 iso-Buten abgetrennt und sogenanntes Raffinat 2 erhalten. Dieses Raffinat-2 wird anschließend einer weiteren Verfahrensstufe (Metathese) zugeführt, in der ein Teil der enthaltenen n-Butene unter Zusatz von Ethylen zu Propylen umgesetzt wird. Ein die Metathese verlassender Butan-reicher C4-Strom (Raffinat-3) wird schließlich zur Verwertung der verbleibenden n-Butene einer weiteren Verfahrensstufe zur oxidativen Dehydrierung zugeführt. Das die oxidative Dehydrierung verlassende Produktgemisch wird in verschiedenen, nicht im Einzelnen beschriebenen Verfahrensstufen aufgetrennt.

Dabei werden unter anderem ein Butadien sowie ein Butan und weitere Verbindungen enthaltender Strom erhalten. Das Verfahren ist für die Herstellung von Butadien ungünstig, da einerseits ein relativ n-Butene-armer Strom (Raffinat-3) eingesetzt wird, während andererseits eine sehr umfangreiche Aufarbeitung zur Abtrennung von Butadien erforderlich ist. Das führt zu hohen spezifischen Investitionskosten.

Ein grundsätzliches Problem der oxidativen Dehydrierung von Butenen zu 1,3-Butadien ist, dass mit steigendem Umsatz die Selektivität für 1,3-Butadien im Allgemeinen immer weiter abnimmt. Bei hohen Umsätzen größer 50%, besonders aber größer 80% und insbesondere größer 90% nimmt die Bildung von Kohlenoxiden, organischen Oxygenaten oder auch koksartigen Komponenten unverhältnismäßig stark zu, was zu erhöhten Kosten durch Verlust von Einsatzstoff, aber auch zu zunehmenden Problemen bei Betrieb oder Aufarbeitung führen kann. Typischerweise liegen im Einsatzstrom mehrere Butenisomere nebeneinander vor, wobei für viele Katalysatorsysteme 1-Buten reaktiver als die 2-Butene ist, so dass also 1-Buten schneller umgesetzt wird als die 2-Butene. Die für einen hohen Umsatz von 2-Butenen notwendigen Reaktionsbedingungen begünstigen entsprechend die Folgechemie von aus 1-Buten relativ rasch gebildetem Butadien zu unerwünschten Nebenprodukten. Für dieses Problem wurden bereits verschiedene Lösungen vorgeschlagen.

So beschreibt die US 2013/0281748 A1 ein Verfahren zur Maximierung der Ausbeute an 1,3-Butadien, bei dem 1-Buten und die 2-Butene parallel zueinander, voneinander getrennt in je einem eigenen Reaktor zu 1,3-Butadien umgesetzt werden, unter Nutzung von unterschiedlich aktiven Katalysatoren. Dieses Verfahren erfordert jedoch die Trennung der Isomere und die doppelte Anzahl der Reaktoren. Eine Umsetzung wird daher durch hohe Investitionskosten erschwert.

In der WO 2014/160825 A1 wird eine Variante des bereits weiter oben angeführten Verfahrens beschrieben, bei dem der herangezogene C4-Strom in einen 1-Buten und iso-Butan reichen Strom sowie einen 2-Butene und n-Butan reichen Strom getrennt werden. Der 1-Buten- und iso-Butan-reiche Strom steht für eine anderweitige Nutzung zur Verfügung. Der 2-Butene und n-Butan reiche Strom wird dagegen der oxidativen Dehydrierung zugeführt. Der Ausgangsstrom aus der oxidativen Dehydrierung wird über verschiedene Aufarbeitungsstufen der Extraktionseinheit zugeführt. In einer Varianten dieses Verfahrens wird nur ein Teilumsatz der 2-Butene erzielt und nicht umgesetzte 2-Butene im Ausgangsstrom nach Abtrennung von 1,3-Butadien in die oxidativen Dehydrierung zurückgeführt. Das abgetrennte 1,3-Butadien wird dagegen über mehrere Aufarbeitungsstufen in die Extraktionseinheit des Crackers zurückgeführt, aus der der ursprünglich eingesetzte C4-Strom entnommen wurde. Dieses komplizierte Verfahren und seine Varianten bergen mehrere Nachteile. So ist es für die Herstellung von Butadien ungünstig, da der eingesetzte Stoffstrom vorwiegend aus sich weitgehend inert verhaltenen Butan und den weniger reaktiven 2-Butene besteht. Bei Varianten des Verfahrens mit Teilumsatz der weniger reaktiven 2-Butene ist eine Abtrennung und Rückführung der nicht umgesetzten 2-Butene zwar technisch machbar, aber auch mit hohen Kosten für Energie oder Investment verbunden. Zugleich ist eine sehr umfangreiche Aufarbeitung zur Abtrennung von 1,3-Butadien erforderlich. Als Möglichkeit der Verarbeitung des zuvor abgetrennten Stroms aus 1-Buten- und iso-Butan wird die Produktion von Polyethylen vorgeschlagen. Für die Produktion von Polyethylen wird 1-Buten aber lediglich als Co-Monomer verwendet, welches zudem vom iso-Butan abgetrennt werden muss. Eine günstige Weiterverarbeitungsmöglichkeit des anfallenden, abgetrennten Stroms wird in WO 2014/160825 A1 nicht beschrieben.

US 4504692 beschreibt einen Prozess zur oxidativen Dehydrierung von Butenen aus einem von iso-Buten befreiten C4-Strom, bei dem nicht umgesetzte n-Butene in die Dehydrierung zurückgefahren werden und dafür Butane abgetrennt werden, so dass zwei Ausgangsströme im Wesentlichen enthaltend Butadien bzw. im Wesentlichen enthaltend Butane erhalten werden. Eine solche Aufspaltung von Butanen und Butenen ist aber apparativ und energetisch aufwendig.

Allgemein stellt sich bei Verfahren, die die Abtrennung eines Teilstroms beinhalten, die Frage nach einer möglichst werthaltigen Nutzung der anfallenden Ströme. Selbiges gilt für Verfahren mit Teilumsatz, bei denen auf eine aufwändige Rückführung der Butene verzichtet wird. Dabei wird die möglichst werthaltige Nutzung eines nach der Abtrennung von 1,3-Butadien anfallenden, Butene enthaltenden C4-Stroms angestrebt.

Es war daher auch eine Aufgabe der vorliegenden Erfindung, ein Verfahren bereitzustellen, bei dem neben einem 1,3-Butadien enthaltenden Stoffstrom ein möglichst werthaltig weiterzuverarbeitender Butene enthaltender Stoffstrom bereitgestellt wird und dieser möglichst werthaltig weiterverarbeitet wird, um die Wirtschaftlichkeit des Verfahrens insgesamt durch möglichst geringe Invest- oder Energiekosten möglichst günstig zu gestalten.

WO 2004/039757 beschreibt ein Verfahren zur Herstellung von Buten-Oligomeren. Dabei wird ein von Butadien befreiter C4-Kohlenwasserstoffstrom aus einer FCC-Anlage enthaltend Butene und Butane eingesetzt. Eine Dehydrierung wird nicht beschrieben.

WO 2006/089956 beschreibt ein Verfahren zur Herstellung von Ethen oder Propen durch Olefin-Metathese eines C4-Kohlenwasserstoff-Einsatzstroms. Der C4-Einsatzstrom kann aus einem C4-Kohlenwasserstoffstrom aus einer FCC-Anlage gewonnen werden, indem dieser durch Extraktivdestillation praktisch von Butadien befreit wird, wobei ein Raffinat I erhalten wird, das 0 bis 5 Gew.-% 1-Buten enthält. Eine Extraktivdestillation zur Abtrennung von Butadien ohne vorherige Anreicherung von Butadien ist bei typischen Strömen aus einer FCC-Anlage (< 1 Gew.-% Butadien) jedoch unrentabel.

US5324419 beschreibt ein Verfahren zum Betrieb einer FCC-Anlage zur Minimierung der Butadien-Ausbeute im C4-Schnitt, da der Gehalt an Butadien im C4-Produkt-Schnitt einer FCC-Anlage in der Regel zu gering ist, um wirtschaftlich sinnvoll extrahiert werden zu können. Allerdings stört Butadien in den meisten Folgeschritten, weswegen es regelmäßig durch selektive Hydrierung entfernt wird. Da hierzu Wasserstoff verbraucht wird, kann es sinnvoll sein, die Butadienbildung in der FCC-Anlage so gering wie möglich zu halten.

US 2009/0030251 A1 beschreibt ein Verfahren zur Herstellung von Alkylaten aus gesättigten und olefinischen C4-Nebenprodukten eines Crackers. Die C4-Kohlenwasserstoff-Einsatzströme können ursprünglich aus einer FCC-Anlage stammen, wobei die Ströme durch Hydrierung von 1,3-Butadien im Wesentlichen befreit werden und das Verhältnis von 2-Buten zu 1-Buten durch Isomerisierung von 1-Buten zu 2-Buten erhöht wird.

Verfahren zur Alkylierung von Isobutan und n-Butenen, wie sie in Raffinerien von Bedeutung sind, werden auch von Corma und Martinez (Catal. Rev. - Sci.Eng., 35(4). 483-570, 1993) sowie von Albright (Ind. Eng. Chem. Res. 2009, 48, 1409-1413) beschrieben. Dabei wird deutlich, dass insbesondere bei Verwendung von Schwefelsäure als Katalysator günstigere Produktzusammensetzungen bei Einsatz von 2-Butenen gegenüber 1-Buten erhalten werden können.

CN 101492334 B beschreibt ein Verfahren zur Herstellung von Propylen aus einem C4-Kohlenwasserstoff-Einsatzstrom durch Olefin-Metathese. Der C4-Kohlenwasserstoff-Einsatzstrom kann ursprünglich aus einer FCC-Anlage stammen, wobei der Strom durch Hydrierung von 1,3-Butadien im Wesentlichen befreit wird und das Verhältnis von 2-Butenen zu 1-Buten durch Isomerisierung von 1-Buten zu 2-Buten erhöht wird. Zudem wird Isobuten destillativ abgetrennt.

CN 101492335 B beschreibt ein Verfahren zur Herstellung von Propylen aus einem C4-Kohlenwasserstoff-Einsatzstrom durch Olefin-Metathese. Der C4-Kohlenwasserstoff-Einsatzstrom kann ursprünglich aus einer FCC-Anlage stammen, wobei der Strom durch Extraktivdestillation von Butadien befreit wird und das Verhältnis von 2-Buten zu 1-Buten durch Isomerisierung erhöht wird. Eine Extraktivdestillation zur Abtrennung von Butadien aus typischen FCC-Anlage-Strömen (< 1 Gew.-% Butadien) ohne vorherige Anreicherung von Butadien ist allerdings aufwendig und wirtschaftlich wenig attraktiv.

Aufgabe der Erfindung ist es also, ein insgesamt vorteilhaftes Verfahren zur Herstellung von 1,3-Butadien durch Dehydrierung von n-Butenen aus einer FCC-Anlage bereitzustellen, bei dem die in dem Einsatzgasstrom der Dehydrierung enthaltenen Kohlenwasserstoffe optimal stofflich verwertet werden.

Gelöst wird die Aufgabe durch ein Verfahren zur Herstellung von 1,3-Butadien aus n-Butenen mit den Schritten:
A) Bereitstellung eines Butane, 1-Buten, 2-Buten und iso-Buten sowie gegebenenfalls 1,3-Butadien enthaltenden Einsatzgasstromes a aus einer FCC-Anlage; in einer bevorzugten Variante des Verfahrens kann die Bereitstellung über eine Einheit zur selektiven Hydrierung erfolgen, welche z.B. zur Umwandlung von Alkinen in Olefine oder zur Umwandlung von Diolefinen in Olefine dient;
B) Abtrennung von iso-Buten aus dem Einsatzgasstrom a, wobei ein Butane, 1-Buten und 2-Butene sowie gegebenenfalls 1,3-Butadien enthaltender Stoffstrom b erhalten wird;
C) Einspeisung des Butane, 1-Buten und 2-Buten enthaltenden Stoffstroms b und gegebenenfalls eines sauerstoffhaltigen Gases sowie gegebenenfalls Wasserdampf in mindestens eine Dehydrierzone und Dehydrierung von 1-Buten und 2-Buten zu 1,3-Butadien, wobei ein Produktgasstrom c enthaltend 1,3-Butadien, Butane, 2-Buten, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird;
D) Abkühlung und Kompression des Produktgasstroms c, wobei mindestens ein wässriger Kondensatstrom d1 und ein Gasstrom d2 enthaltend 1,3-Butadien, Butane, 2-Butene, Wasserdampf, gegebenenfalls Sauerstoff, leicht siedende Kohlenwasserstoffe, gegebenenfalls Kohlenstoffoxide und gegebenenfalls Inertgase erhalten wird;
Ea) Abtrennung von nicht kondensierbaren und leicht siedenden Gasbestandteilen umfassend leicht siedende Kohlenwasserstoffe, gegebenenfalls Sauerstoff, gegebenenfalls Kohlenstoffoxide und gegebenenfalls Inertgase als Gasstrom e2 aus dem Gasstrom d2 durch Absorption der C₄-Kohlenwasserstoffe umfassend 1,3-Butadien, Butane und 2-Butene in einem Absorptionsmittel, wobei ein mit C₄-Kohlenwasserstoffen beladener Absorptionsmittelstrom und der Gasstrom e2 erhalten werden, und
Eb) anschließende Desorption der C₄-Kohlenwasserstoffe aus dem beladenen Absorptionsmittelstrom, wobei ein C4-Kohlenwasserstoffstrom e1 erhalten wird;
F) Auftrennung des C₄-Kohlenwasserstoffstroms e1 durch Extraktivdestillation mit einem für 1,3-Butadien selektiven Lösungsmittel in einen 1,3-Butadien und das selektive Lösungsmittel enthaltenden Stoffstrom f1 und einen Butane und 2-Butene enthaltenden Stoffstrom f2,
dadurch gekennzeichnet, dass
in Schritt C) mindestens 90%, bevorzugt mindestens 95% und insbesondere bevorzugt mindesten 99% des in Strom b enthaltenen 1-Buten umgesetzt werden, und in Schritt F ein Butane und 2-Butene enthaltender Produktstrom bereitgestellt wird.

In einer bevorzugten Ausführungsform wird der in Schritt F) erhaltene, Butane und 2-Buten enthaltende Stoffstrom f2 weiter umgesetzt gemäß einem oder mehreren der Schritte G1), G2) oder G3) durch
G1) Alkylierung von 2-Butenen mit Butanen zu Isooctanen;
G2) Olefin-Metathese von 2-Butenen mit Ethen zu Propen;
G3) Oligomerisierung von 2-Buten.

Der erhaltene Stoffstrom f2 kann bei jedem der möglichen Schritte G1), G2) oder G3) gegebenenfalls noch vorbehandelt werden kann. Z.B. kann gegebenenfalls noch in geringen Mengen enthaltenes Butadien z.B. durch selektive Hydrierung entfernt werden.

Erfindungsgemäß wird ein von Isobuten befreiter C₄-Kohlenwasserstoffstrom aus einer FCC-Anlage, enthaltend Butane und n-Butene (1-Buten und 2-Buten) und gegebenenfalls geringe Mengen an 1,3-Butadien, einer Dehydrierstufe zugeführt.

Der aus dem Produktgasgemisch der Dehydrierstufe abgetrennte C₄-Schnitt (vorwiegend Butane, 1,3-Butadien und 2-Buten) wird einer Butadien-Extraktion zugeführt, so dass der verbleibende Strom im Wesentlichen Butane (n-Butan und iso-Butan) und 2-Buten (cis- und trans-2-Buten) enthält. Die Dehydrierstufe kann dabei, insbesondere im Falle einer oxidativen Dehydrierung, so durchgeführt werden, dass 1-Buten praktisch quantitativ umgesetzt wird, jedoch nicht umgesetztes 2-Buten im Produktgasstrom der Dehydrierung verbleibt. Die Stufen C), D), E) und F) des erfindungsgemäßen Verfahrens können in einer typischen Raffinerie-Infrastruktur die Hydro-Isomerisierungseinheit zur Anreicherung von 2-Butenen gegenüber 1-Buten und die Einheit zur Entfernung von Oxygenaten ersetzen. Eine Hydrierungseinheit kann vorgesehen werden, um einen geeigneten Einsatzgasstrom für die Dehydrierung bereitzustellen, beispielsweise um Acetylene, Alkine oder 1,2-Butadien zu entfernen. Butane verhalten sich in der Dehydrierstufe weitgehend inert. Oxygenate, wie beispielsweise Formaldehyd, Ameisensäure, Acetyldehyd, Essigsäure, Acrolein, Acrylsäure, Propionaldehyd, Proprionsäure, Methacrolein, Methacrylsäure, Crotonaldehyd, Crotonsäure, Methylvinylketon, Furan, Maleinsäureanhydrid, Styrol, Benzaldehyd, Benzoesäure, Phthalsäureanhydrid, Fluorenon, Anthrachinon und Butyraldehyd, werden im erfindungsgemäßen Verfahren vor allem in den Stufen D), E) und F) abgetrennt. Sollte die Trennleistung für Oxygenate nicht ausreichend sein oder sollte einen noch höhere Trennleistung für das weitere Verfahren, besonders für die Schritte G1), G2) und G3), vorteilhaft sein, so können in einer bevorzugten Variante weitere Prozesschritte nach der Stufe C) oder späteren Stufen durchgeführt werden, welche die Konzentration der Oxygenate im Stoffstrom f2 weiter verringern.

Somit werden mit dem erfindungsgemäßen Verfahren die nachstehenden Vorteile realisiert:
(i) Ein Teil des aus der FCC-Anlage stammende C₄-Kohlenwasserstoffgemisch wird zu 1,3-Butadien veredelt.
(ii) Die Dehydrierstufe kann in einem Bereich mit relativ niedrigen Buten-Umsätzen und relativ hohen Selektivitäten betrieben werden, wobei neben Butadien ein weiterer Ausgangsstrom enthaltend im Wesentlichen 2-Butene und Butane bereitgestellt wird, der insbesondere auch in Bezug auf die enthaltenen 2-Butene sehr werthaltig verwertet werden kann, ohne dass diese abgetrennt oder weghydriert werden müssen.
(iii) Der nach Abtrennung von 1,3-Butadien durch Extraktivdestillation verbleibende C₄-Kohlenwasserstoffstrom f2 ist reich an 2-Buten und Butanen und arm an 1-Buten. Er eignet sich für eine werthaltige Nutzung, insbesondere für eine weitere Umsetzung
   a. durch Alkylierung von 2-Buten mit Butanen zu Isooctanen,
   b. durch Olefin-Metathese mit Ethylen zu Propylen,
   c. durch Oligomerisierung zu Buten-Oligomeren, insbesondere zu C₈- und C₁₂-Olefinen; diese können optional wieder in die FCC-Anlage zurückgeführt werden.

In einer Stufe A) wird ein Einsatzgasstrom a enthaltend n-Butene und iso-Buten, sowie gegebenenfalls geringe Mengen 1,3-Butadien, aus einer FCC-Anlage bereitgestellt. In einer bevozugten Variante werden der gegebenenfalls vorhandene geringe Anteil an 1,3-Butadien und gegebenenfalls auch in geringen Anteilen vorhandene Diene oder Alkine durch selektive Dehydrierung entfernt.

Typischerweise enthält die als Einsatzgasstrom a bereitgestellte C₄-Produktfraktion aus einer FCC-Anlage, je nach "Crackschärfe" ca. 0,2 bis 1,0 Vol.%1,3-Butadien, ca. 10 bis 20 Vol.% 1-Buten, 10 bis 30 Vol.-% 2-Buten (cis- und trans-2-Buten), ca. 15 bis 25 Vol.-% iso-Buten, 5 bis 15 Vol.-% n-Butan und 25 bis 40 Vol.% iso-Butan.

**Tabelle 1. Typische Zusammensetzung von Komponenten (in wt-%) von C4-Pordukfraktionen einer FCC-Anlage bei mittlerer "Crackschärfe" gemäß Ullmann's Encyclopedia of Industrial Chemistry, Published Online: 31 JAN 2014 Butene**

| Komponente | FCC | Steam-Cracker |
|---|---|---|
| iso-Butan | 37 | 2 |
| n-Butan | 13 | 6 |
| iso-Buten | 15 | 26 |
| 1-Buten | 12 | 14 |
| trans-2-Buten | 12 | 5 |
| cis-2-Buten | 11 | 4 |
| 1,3-Butadien | <0,5 | 43 |

Die C₄-Produktfraktion aus einer FCC-Anlage zeichnet sich durch einen niedrigen Anteil an 1,3-Butadien und hohe Anteile an 1-Buten, 2-Buten sowie Butanen aus. Im Gegensatz dazu enthält die C₄-Produktfraktion eines Naphtha-Steamcrackers typischerweise einen hohen Anteil an 1,3-Butadien (bis ca. 50 Vol.-%), dafür jedoch nur geringe Mengen an iso-Butan.

Bei dem bekannten FCC-Prozess (vgl. Ullmann's Encyclopedia of Industrial Chemistry, Wiley-VCH Verlag GmbH, Weinheim, Germany, Sixth Edition, 2000 Electronic Release, Chapter Oil Refining, 3.2. Catalytic Cracking) wird der entsprechende Kohlenwasserstoff verdampft und in einem Kurzeitreaktor - dem sogenannten "Riser" - in der Gasphase mit einem pulverförmigen, heterogenen Katalysator bei einer Temperatur von 450 bis 500°C in Kontakt gebracht. Typischerweise wird einer FCC-Einheit eine Fraktion mit einer durchschnittlichen molaren Masse von ca. 200 bis ca. 600 g mol und einem Siedebereich von ca. 340 °C bis ca. 560 °C zugeführt. Geeigent erscheinen daher schweres Gasöl, Vakuumgasöl (VGO), "Deasphalted Oil" (DAO) oder Furfural-Extrakt. Die Katalysator-Partikel werden durch den Kohlenwasserstoffstrom entlang des Risers transportiert und gelangen danach in den Zyklonteil der FCC-Anlage. Dort wird der pulverförmige Katalysator vom gasförmigen Produktstrom des Prozesses abgetrennt und dem Regenerationsteil der FCC-Anlage zugeführt.

Verschiedene Produktströme werden aus dem Rohproduktstrom des FCC-Prozesses in mehreren Aufarbeitungsschritten gewonnen. Im Hauptteil der Produktabtrennung (engl. main fractionator) werden zunächst das slurry oil und das Schweröl (engl. fuel oil) sowie höhersiedende Nebenprodukte (engl. side products) z.B. heavy naphtha und light cycle oil in einer Destillationskolonne aus dem Rohproduktstrom abgetrennt. Der verbleibende Produktstrom wird danach in einen Kondensationsteil (engl. GCU = Gas concentration unit) überführt und darin das FCC-Rohbenzin (engl. Overhead liquid) verflüssigt. Dieser Strom wird in der Flüssiggasabtrennung schließlich in das FCC-Benzin (engl. cracked naphtha), die Flüssiggasfraktion (LPG = liquified petroleum gas) und das verbleibende FCC-Restgas (fuel gas) aufgetrennt. Die Flüssiggasfraktion enthält die hier interessierenden C₄-Kohlenwasserstoffe sowie C3-Kohlenwasserstoffe. Letztere werden meistens in einem weiteren Anlagenteil - der sog. Propylengewinnung (Propylene recovery unit) - von der C4-Fraktion getrennt, wodurch letztendlich die FCC-C4-Fraktion erhalten wird.

Als Katalysator für den FCC-Prozess dienen üblicherweise synthetische kristalline Zeolite, an deren sauren Zentren die Kohlenwasserstoff-Moleküle des Einsatzstoffes katalytisch gespalten werden.

In einem Schritt B) wird iso-Buten aus dem Einsatzgastrom a abgetrennt, wobei ein Butane und n-Butene enthaltender Stoffstrom b erhalten wird. Die Abtrennung erfolgt im Allgemeinen durch Derivatisierung von iso-Buten und nachfolgender Abtrennung des Derivats durch ein thermisches Trennverfahren. Vorzugsweise wird iso-Buten mit Methanol zu Methyl-tert.-butylether (MTBE) oder mit Ethanol zu Ethyl-tert.-butylether (ETBE) derivatisiert und das Derivat anschließen destillativ abgetrennt. Derartige Verfahren sind dem Fachmann prinzipiell bekannt und beispielsweise in US 7,932,428 B2 beschrieben. Die Derivatisierung, beispielsweise durch Veretherung zu MTBE oder ETBE und destillativer Abtrennung des Derivats, kann beispielsweise in einem gemeinsamen Verfahrensschritt in einer Reaktivdestillationskolonne durchgeführt werden.

In einem Schritt C) werden der Butane und n-Butene enthaltende Stoffstrom b und gegebenenfalls ein sauerstoffhaltiges Gas und gegebenenfalls Wasserdampf in mindestens eine Dehydrierzone eingespeist und werden die n-Butene zu 1,3-Butadien dehydriert, wobei ein Produktgasstrom c enthaltend 1,3-Butadien, Butane, nicht umgesetzte n-Butene, insbesondere 2-Buten, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird. Der Schritt C) wird so durchgeführt, dass das in dem Stoffstrom b enthaltenen 1-Buten zu mindestens 90%, bevorzugt mindestens 95% und insbesondere bevorzugt mindesten 99%, umgesetzt wird.

Der Schritt C) kann als nicht-oxidative Dehydrierung durchgeführt werden. In diesem Fall werden der Butane und n-Butene enthaltende Stoffstrom b und Wasserdampf in die mindestens eine Dehydrierzone eingespeist und werden n-Butene zu 1,3-Butadien dehydriert, wobei ein Produktgasstrom c enthaltend 1,3-Butadien, nicht umgesetzte n-Butene, insbesondere 2-Buten, Wasserdampf, leicht siedende Kohlenwasserstoffe und hochsiedende Nebenkomponenten erhalten wird.

Bevorzugt wird Schritt C) als oxidative Dehydrierung (Oxidehydrierung, ODH) durchgeführt. In diesem Fall werden der Butane und n-Butene enthaltende Stoffstrom b und ein sauerstoffhaltiges Gas in die mindestens eine Dehydrierzone eingespeist und werden n-Butene zu 1,3-Butadien dehydriert, wobei ein Produktgasstrom c enthaltend 1,3-Butadien, Butane, nicht umgesetzte n-Butene, insbesondere 2-Buten, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (la) auf:

Mo₁₂BiₐFe_{b}Co_{c}Ni_{d}CrₑX¹_{f}X²_{g}O_{y} (Ia),

mit
X¹ = Si, Mn und/oder AI,
X² = Li, Na, K, Cs und/oder Rb,
0,2 ≤ a ≤ 1,
0,5 ≤ b ≤ 10,
0 ≤ c ≤ 10,
0 ≤ d ≤ 10,
2 ≤ c + d ≤ 10
0 ≤ e ≤ 2,
0 ≤ f ≤ 10
0 ≤ g ≤ 0,5
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K. Besonders bevorzugt ist ein weitgehend Cr(VI)-freier Katalysator.

Ganz besonders bevorzugt werden oxidative Dehydrierungen unter Verwendung von Katalysatoren enthaltend Oxide von Bismuth und Molybdän, wie beispielsweise in der US 2012/0130137A1 beschrieben, durchgeführt. Sie sind besonders dazu geeignet, einen praktisch vollständigen Umsatz von 1-Buten, neben einem Teilumsatz von 2-Butenen, einzustellen. Als eine mögliche Quelle für den C4-Gasstrom wird in der US2012/0130137A1 unter anderem ein FCC-Cracker genannt.

Zur Durchführung der oxidativen Dehydrierung (ODH) bei hohem Gesamtumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigem Gas und gegebenenfalls zusätzlichem Inertgas, Methan oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen oder Salzgemischen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Auf Grund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1-150 °C, bevorzugt zwischen 10-100 °C und besonders bevorzugt zwischen 20-80 °C. Die Temperatur am Ende des Katalysatorbettes liegt im Allgemeinen zwischen 0-100 °C, vorzugsweise zwischen 0,1-50 °C, besonders bevorzugt zwischen 1-25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Wenn einzelne Rohre mit Thermohülse ausgestattet werden sollen, wird der Druckverlust über diese Rohre bevorzugt so eingestellt, dass er dem der anderen Rohre weitgehend entspricht.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im ODH-Reaktor eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial und/oder geträgerten Schalenkatalysatoren bestehen.

Für viele Katalysatorsysteme sind 1-Butene reaktiver als 2-Butene, so dass sie im Reaktor unter gegebenen Bedingungen bevorzugt abreagieren, bevor 2-Butene vollständig umgesetzt werden. Der Gesamt-Umsatz an Butenen ist unter gegebenen Reaktionsbedingungen damit auch von dem genauen Verhältnis von 1- zu 2-Butenen abhängig. Der Gesamtumsatz von Butenen in einem technischen Verfahren kann vom Fachmann auf Grundlage experimenteller Ergebnisse mit einem bestimmten Katalysator und ggf. entsprechender Modellierung eingestellt werden. Ein bestimmter Zielumsatz kann durch verschiedene Stellgrößen, beispielsweise Salzbadtemperatur, Durchmesser und Länge der Reaktorrohre und Länge bzw. Volumen der Katalysatorschüttungen, ggf. Verdünnung der Katalysatoren mit Inertmaterial oder Belastung der Reaktoren mit Feed-Strom usw. eingestellt werden. Bei einem Umsatz der Gesamtbutene von mindesten 48% Prozentpunkten über dem Anteil von 1-Buten an den Butenen ist regelmäßig von einem praktisch vollständigen Umsatz von 1-Buten (größer 98%) auszugehen. Zur Steuerung kann auch eine entsprechende Analyse des Reaktorausgangsstroms herangezogen werden.

Der die oxidative Dehydrierung verlassende Produktgasstrom c enthält neben Butadien im Allgemeinen noch Butane und nicht umgesetzte n-Butene, Sauerstoff sowie Wasserdampf. Erfindungsgemäß wird 1-Buten nahezu vollständig und 2-Buten nur unvollständig umgesetzt. Als weitere Komponenten enthält der Strom c im Allgemeinen Kohlenmonoxid, Kohlendioxid, Inertgase (hauptsächlich Stickstoff), gegebenenfalls leichtsiedende Kohlenwasserstoffe wie Methan, Ethan, Ethen, Propan und Propen, Butan und iso-Butan, sowie sauerstoffhaltige Kohlenwasserstoffe, sogenannte Oxygenate. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Im Allgemeinen enthält der Produktgasstrom c noch maximal 10%, bevorzugt maximal 5%, insbesondere weniger als 1% des im Beschickungsgasgemisch der Oxidehydrierung (Strom b) enthaltenen 1-Buten. Im Allgemeinen enthält der Produktgasstrom c noch mindestens 10%, bevorzugt mindestens 15% des im Beschickungsgasgemisch der Oxidehydrierung (Strom b) enthaltenen 2-Buten. In einem Schritt D) wird der Produktgasstrom c abgekühlt und komprimiert, wobei mindestens ein wässriger Kondensatstrom d1 und ein Gasstrom d2 enthaltend 1,3-Butadien, Butane, n-Butene, Wasserdampf, Sauerstoff, leicht siedende Kohlenwasserstoffe, gegebenenfalls Kohlenstoffoxide und gegebenenfalls Inertgase erhalten wird.

Im Allgemeinen umfasst Schritt D) die Schritte:
Da) Abkühlung des Produktgasstroms c durch Inkontaktbringen mit einem Kühlmittel und Kondensation zumindest eines Teils der hochsiedenden Nebenkomponenten;
Db) Kompression des verbleibenden Produktgasstroms c in mindestens einer Kompressionsstufe, wobei mindestens ein wässriger Kondensatstrom d1 und ein Gasstrom d2 enthaltend 1,3-Butadien, Butane, n-Butene, Wasserdampf, Sauerstoff, leicht siedende Kohlenwasserstoffe, gegebenenfalls Kohlenstoffoxide und gegebenenfalls Inertgase erhalten wird.

Im Allgemeinen wird in der Abkühlstufe Da) ein wässriges Kühlmittel oder ein organisches Lösungsmittel verwendet.

Vorzugsweise wird in der Abkühlstufe Da) ein organisches Lösungsmittel verwendet. Diese weisen im Allgemeinen ein sehr viel höheres Lösungsvermögen für die hochsiedenden Nebenprodukte, die in den dem ODH-Reaktor nachgelagerten Anlageteilen zu Ablagerungen und Verstopfungen führen können, auf als Wasser oder alkalisch-wässrige Lösungen. Bevorzugte als Kühlmittel eingesetzte organische Lösungsmittel sind aromatische Kohlenwasserstoffe, beispielsweise Toluol, o-Xylol, m-Xylol, p-Xylol, Diethylbenzole, Trietylbenzole, Diisopropylbenzole, Triisopropylbenzole und Mesitylen oder Gemische daraus. Besonders bevorzugt ist Mesitylen.

Nachstehende Ausführungsformen sind bevorzugte bzw. besonders bevorzugte Varianten des erfindungsgemäßen Verfahrens:
Die Stufe Da) wird mehrstufig in Stufen Da1) bis Dan), bevorzugt zweistufig in zwei Stufen Da1) und Da2) durchgeführt. Dabei wird besonders bevorzugt zumindest ein Teil des Lösungsmittels nach Durchlaufen der zweiten Stufe Da2) als Abkühlmittel der ersten Stufe Da1) zugeführt.

Die Stufe Db) umfasst im Allgemeinen mindestens eine Kompressionsstufe Dba) und mindestens eine Abkühlstufe Dbb). Bevorzugt wird in der mindestens einen Abkühlstufe Dbb) das in der Kompressionsstufe Dba) komprimierte Gas mit einem Abkühlmittel in Kontakt gebracht. Besonders bevorzugt enthält das Abkühlmittel der Abkühlstufe Dbb) das gleiche organische Lösungsmittel, das in der Stufe Da) als Abkühlmittel verwendet wird. In einer insbesonders bevorzugten Variante wird zumindest ein Teil dieses Abkühlmittels nach Durchlaufen der mindestens einen Abkühlstufe Dbb) als Abkühlmittel der Stufe Da) zugeführt.

Bevorzugt umfasst die Stufe Db) mehrere Kompressionsstufen Dba1) bis Dban) und Abkühlstufen Dbb1) bis Dbbn), beispielsweise vier Kompressionsstufen Dba1) bis Dba4) und vier Abkühlstufen Dbb1) bis Dbb4).

Anschließend werden in einem Absorptionsschritt Ea) die nicht kondensierbaren und leicht siedenden Gasbestandteile umfassend leicht siedende Kohlenwasserstoffe, gegebenenfalls Sauerstoff, gegebenenfalls Kohlenstoffoxide und gegebenenfalls Inertgase als Gasstrom e2 aus dem Gasstrom d2 durch Absorption der C₄-Kohlenwasserstoffe umfassend 1,3-Butadien, Butane und n-Butene in einem Absorptionsmittel abgetrennt, wobei ein mit C₄-Kohlenwasserstoffen beladener Absorptionsmittelstrom und der Gasstrom e2 erhalten werden, und in einem anschließende Desorptionsschritt Eb) die C₄-Kohlenwasserstoffe aus dem beladenen Absorptionsmittelstrom desorbiert, wobei ein C4-Kohlenwasserstoffstrom e1 erhalten wird.

Der sauerstoffhaltige Gasstrom e2 wird im Allgemeinen nach Abtrennung eines Purge-Gasstroms als Kreislaufstrom in die oxidative Dehydrierung zurückgeführt. Der Purge-Strom wird im Allgemeinen einer Abgasbehandlung zugeführt und kann beispielsweise in einer Fackel, durch katalytische Nachverbrennung oder in einem Prozessbrenner verbrannt werden.

Der Gasstrom e2 kann auch einer Abgasbehandlung zugeführt werden. Bevorzugt umfasst Schritt Ea) die Schritte Ea1), Ea2) und Eb):
Ea1) Absorption der C₄-Kohlenwasserstoffe umfassend 1,3-Butadien, n-Butene und Butane in einem hochsiedenden Absorptionsmittel, wobei ein mit C₄-Kohlenwasserstoffen beladener Absorptionsmittelstrom und der Gasstrom f2 erhalten werden,
Ea2) Entfernung von Sauerstoff aus dem mit C₄-Kohlenwasserstoffen beladenen Absorptionsmittelstrom aus Schritt Ea1) durch Strippung mit einem nicht kondensierbaren Gasstrom, und
Eb) Desorption der C₄-Kohlenwasserstoffe aus dem beladenen Absorptionsmittestrom, wobei ein C₄-Kohlenwasserstoffstrom f1 erhalten wird, der im Wesentlichen aus C₄-Kohlenwasserstoffen besteht und weniger als 100 ppm Sauerstoff umfasst.

In einer Ausführungsform ist das in Schritt Ea) eingesetzte hochsiedende Absorptionsmittel ein aromatisches Kohlenwasserstofflösungsmittel, besonders bevorzugt das in Schritt Da) eingesetzte aromatische Kohlenwasserstofflösungsmittel, insbesondere Mesitylen. Verwendet werden können auch Diethylbenzole, Triethylbenzole, Diisopropylbenzole und Triisopropylbenzole.

Die Absorptionsstufe kann in jeder beliebigen, dem Fachmann bekannten geeigneten Absorptionskolonne durchgeführt werden. Die Absorption kann durch einfaches Durchleiten des Produktgasstroms d2 durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der 1,3-Butadien, Butane, n-Butene und die leichtsiedenden und nicht kondensierbaren Gasbestandteile enthaltende Gasstrom d2 zugeführt. Im oberen Bereich der Absorptionskolonne wird das hochsiedende Absorptionsmittel aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

In einer bevorzugten Ausführungsform in der Absorptionsstufe Ea1) wird das gleiche Lösungsmittel wie in der Abkühlstufe Da) eingesetzt.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. Bevorzugt sind aromatische Kohlenwasserstoffe, besonders bevorzugt Toluol, o-Xylol, p-Xylol und Mesitylen, oder Gemische daraus. Verwendet werden können auch Diethylbenzol, Triethylbenzol, Diisopropylbenzol und Triisopropylbenzol.

Am Kopf der Absorptionskolonne wird ein Gasstrom e2 abgezogen, der im Wesentlichen Sauerstoff, leicht siedende Kohlenwasserstoffe (Methan, Ethan, Ethen, Propan, Propen), gegebenenfalls C₄-Kohlenwasserstoffe (Butan, Butene, 1,3-Butadien), gegebenenfalls Inertgase, gegebenenfalls Kohlenstoffoxide und gegebenenfalls noch Wasserdampf enthält.

Am Sumpf der Absorptionskolonne können in einer weiteren Kolonne durch die Spülung mit einem Gas Reste von im Absorptionsmittel gelöstem Sauerstoff ausgetragen werden. Der verbleibende Sauerstoffanteil ist vorzugsweise so klein, dass der die Desorptionskolonne verlassende und Butan, Butene sowie 1,3-Butadien enthaltende Strom nur noch maximal 100 ppm Sauerstoff enthält. Durch die Begrenzung des Sauerstoffgehalts wird die Bildung von Peroxiden und von voluminösen Polymeren ("Popcorn") vermieden.

Das Ausstrippen des Sauerstoffs in Schritt Eb) kann in jeder beliebigen, dem Fachmann bekannten geeigneten Kolonne durchgeführt werden. Das Strippen kann durch einfaches Durchleiten von nicht kondensierbaren Gasen, vorzugsweise nicht oder nur schwach im Absorptionsmittelstrom absorbierbare Gase wie Methan, durch die beladene Absorptionslösung erfolgen. Mit ausgestrippter C₄-Kohlenwasserstoff wird im oberen Teil der Kolonne zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil gleich ist, kann diese direkte Kopplung erfolgen. Geeignete Stippkolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

In Schritt F) wird der C₄-Kohlenwasserstoffstrom e1 durch Extraktivdestillation mit einem für Butadien selektiven Lösungsmittel in einen 1,3-Butadien und das selektive Lösungsmittel enthaltenden Stoffstrom f1 und einen Butane und 2-Buten enthaltenden Stoffstrom f2 aufgetrennt.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Der Butane und 2-Buten enthaltenden Stoffstrom f2 enthält im Allgemeinen hauptsächlich Butane (iso- oder n-Butan). Bei den enthaltenen n-Butenen handelt es sich in der Regel, vor allem dann, wenn die vorausgehende Dehydrierung als Oxidehydrierung ausgeführt wurde, hauptsächlich um cis- oder trans-2-Buten mit nur noch geringen Mengen von 1-Buten. Der Strom f2 unterscheidet sich also von dem Strom b des erfindungsgemäßen Verfahrens durch einen deutlich höheren Anteil an Butanen und einen deutlich höheren Anteil an 2-Buten gegenüber 1-Buten. Bei den im Strom f2 verbliebenen n-Butenen handelt es sich in der Regel ganz überwiegend um 2-Butene. Gegebenenfalls im Strom b noch vorhandene, nicht vollständig abgetrennte kleinere Mengen an iso-Buten werden in der Oxidehydrierung weitgehend unselektiv umgesetzt.

Der Stoffstrom f2 fällt typischerweise mit einem Druckniveau von 4-6 bar an. Dieses Druckniveau entspricht typischen Drucken im Falle einer H₂SO₄-katalysierten Alkylierung zu Isooctan. Im Falle einer HF-katalysierten Alkylierung ist ein Durckniveau von 8-20 bar üblich.

Der am Sumpf der Extraktivdestillationskolonne gewonnene Stoffstrom f1 enthält im Allgemeinen das Extraktionsmittel, 1,3-Butadien und in geringen Anteilen 2-Buten und Butane.

Der Strom f2 wird erfindungsgemäß gemäß in einem oder mehreren der Schritte G1), G2) oder G3) durch
G1) Alkylierung von 2-Buten mit Butanen zu Isooctanen;
G2) Olefin-Metathese von 2-Buten mit Ethen zu Propen;
G3) Oligomerisierung von 2-Buten;
weiter umgesetzt.

In die Alkylierungsstufe G1) werden der Strom f2 und gegebenenfalls weitere Butane eingespeist. Die Alkylierung wird im Allgemeinen bei Temperaturen von 40 bis 120 °C und Drücken von 3,5 bar und 42 bar durchgeführt. Geeignete Alkylierungskatalysatoren umfassen Schwefelsäure und Fluorwasserstoffsäure sowie Feststoffsäuren wie chloriertes Aluminiumoxid, Aluminosilikate und Aluminophosphate.

Es wird ein Gemisch aus Isooctanen (sogenanntes Alkylat-Benzin) erhalten.

Die zugrunde liegende Chemie lässt sich vereinfachend mit zwei Teilreaktionsgleichungen beschreiben:

*t*-C₄H₉⁺ + 2-Buten → *i*-C₈H₁₇⁺

*i*-C₈H₁₇⁺ + *i*-C₄H₁₀ → *i*-C₈H₁₈ + *t*-C₄H₉⁺

Das gewünschte Produkt, das höher verzweigte Trimethylpentan, ist bei Reaktion von 2-Butenen stark begünstigt, wohingegen bei Reaktion mit 1-Buten in deutlich größerer Menge, das weniger verzweigte Dimethylhexan gebildet wird. Die Anreicherung von 2-Butenen gegenüber 1-Buten im Eingangsstrom ist daher anzustreben.

In die Metathesestufe G2) werden der Strom f2 und Ethen eingespeist, wobei man pro Mol im Strom f2 enthaltenes 2-Buten (cis- und trans-2-Buten) 0,5 bis 2, vorzugsweise 0,9 bis 1,2 mol Ethen einsetzt. Der Strom f2 kann vor der Einspeisung in die Methathesestufe optional durch selektive Hydrierung von gegebenenfalls verbliebenen Restemengen an 1,3-Butadien oder anderen Diolefinen oder Alkinen befreit werden.

Für die Metathese kommen prinzipiell verschiedene Katalysatortypen in Frage, z.B. : a) Rhenium-haltige Katalysatoren, die bei Temperaturen im Bereich von 0 bis 150 °C, bevorzugt im Bereich von 35 bis 110 °C betrieben werden und b) Wolfram-haltige, Renium-freie Katalysatoren, die in der Gasphase bei Temperaturen von 200 bis 600 °C, bevorzugt von 220 bis 450 °C betrieben werden.

Die Rhenium-haltigen Katalysatoren enthalten bevorzugt wenigstens 1 Gew.-% Rhenium in oxidischer Form auf einem Träger, der zu wenigstens 75 Gew.-% aus einem hochoberflächigem Aluminiumoxid, ganz besonders bevorzugt gamma-Aluminiumoxid, besteht. Insbesondere bevorzugt sind Katalysatoren die einen Rhenium-Gehalt zwischen 5 und 12 Gew.-% aufweisen und auf reinem gamma-Al₂O₃ geträgert sind. Die Katalysatoren können zur Steigerung der Aktivität auch noch zusätzlich Dotierstoffe enthalten, beispielsweise Oxiden von Nb, Ta, Zr, Ti, Fe, Mn, Si, Mo, W, Phosphat oder Sulfat. Die Katalysatoren haben Oberflächen von wenigstens 50 m²/g, bevorzugt wenigstens 100 m²/g, und ein Porenvolumen von wenigstens 0,3 ml/g, bevorzugt wenigstens 0,4 ml/g. Geeignete Renium-haltige Katalysatoren sind beispielsweise in DE-A-102004009804, DE-A-102004009805 oder DE-A-102004009803 beschrieben.

Geeignete Wolfram-haltige, Rhenium-freie Katalysatoren enthalten bevorzugt wenigstens 3 Gew.-% Wolfram, zumindest teilweise in oxidischer Form, auf einem Träger, ausgewählt aus der Gruppe Aluminiumoxide, Alumosilikate, Zeolithe oder bevorzugt SiO₂. Die Katalysatoren weisen bevorzugt eine Oberfläche von wenigstens 50 m²/g und ein Porenvolumen von wenigstens 0,3 ml/g, besonders bevorzugt wenigstens 0,5 ml/g, auf. Die Aktivität bzw. Isomerisierungsaktivität kann durch geeignete Dotierungen verändert werden, beispielsweise mit Alkali- und Erdalkaliverbindungen, TiO₂, ZrO₂, HfO₂, oder mit Verbindungen oder Elementen aus der Gruppe bestehend aus Ag, Sb, Mn, W, Mo, Zn und Si.

Es ist dem Fachmann bekannt, dass alle Arten von Metathesekatalysatoren regelmäßig oxidativ regeneriert werden müssen. Hierzu wird entweder ein Aufbau mit Festbetten und wenigstens zwei Reaktoren gewählt, von denen sich immer wenigstens ein Reaktor im Regenerationsmodus befindet, oder es kann alternativ ein Wanderbettverfahren ausgeübt werden, bei dem deaktivierter Katalysator ausgeschleust und extern regeneriert wird.

Der in der Metathesestufe gebildete Kohlenwasserstoffstrom wird im Allgemeinen durch allgemein bekannte Destillationsverfahre, ggf. in mehreren Stufen, aufgetrennt.

In die Oligomerisierungsstufe G3) wird der Strom f2 eingespeist. Bevorzugt wird 2-Buten zu Octenen und Dodecenen oligomerisiert. Dabei werden im Allgemeinen Nickelkatalysatoren eingesetzt.

Octene bzw. Dodecene stellen wertvolle Zwischenprodukte dar, die insbesondere durch Hydroformylierung und nachfolgende Hydrierung zu Nonanol bzw Tridecanol umgesetzt werden können.

Als Ni-Katalysatoren kommen vor allem solche Nickel enthaltende Katalysatoren zum Einsatz, die bekanntermaßen eine geringe Oligomeren-Verzweigung bewirken, wie in DE 43 39 713 und WO 01/37989 und in den darin zum Stand der Technik zitierten Literaturstellen beschrieben. Besonders bevorzugt sind Katalysatoren, die sowohl Schwefel als auch Ni als Aktivkomponente enthalten.

Ganz besonders bevorzugt werden Katalysatoren kombiniert, die sich in ihrem S : Ni-Verhältnis unterscheiden. Mit Vorteil wird in der vorderen Reaktionsstufe ein Katalysator mit einem S : Ni-Verhältnis < 0,5 mol/mol, bevorzugt ein Katalysator gemäß WO 01/37989 oder DE 43 39 713 und in der hinteren Reaktionsstufe ein Katalysator mit einem S : Ni-Verhältnis > 0,5 mol/mol, bevorzugt ein Katalysator gemäss EP 272970, US 3959400, FR 2641477 oder US 4511750 mit einem S : Ni-Verhältnis > 0,8, besonders bevorzugt 1,0, eingesetzt.

Die oben genannten Katalysatoren kommen z. B. in Verfahren zum Einsatz, wie sie z. B. in WO 99/25668 und WO 01/72670 beschrieben sind und auf die hiermit ausdrücklich Bezug genommen wird.

Ist der Ni-Katalysator im Reaktor in mehreren Festbetten angeordnet, so kann der Feed aufgeteilt und an mehreren Stellen, z. B. vor einem ersten Festbett in Fließrichtung des Reaktionsgemisches und/oder zwischen einzelnen Ni-Katalysatorfestbetten, in den Reaktor eingeleitet werden. Bei Verwendung einer Reaktorkaskade beispielsweise ist es möglich, den Feed vollständig dem ersten Reaktor der Kaskade zuzuführen oder ihn über mehrere Zuleitungen auf die einzelnen Reaktoren der Kaskade, wie für den Fall des Einzelreaktors beschrieben, zu verteilen.

Die Oligomerisierungsreaktion findet in der Regel bei Temperaturen von 30 bis 280, vorzugsweise von 30 bis 190 und insbesondere von 40 bis 130 C und einem Druck von in der Regel 1 bis 300, vorzugsweise von 5 bis 100 und insbesondere von 10 bis 50 bar statt. Der Druck wird dabei zweckmäßigerweise so gewählt, dass der Feed bei der eingestellten Temperatur überkritisch und insbesondere flüssig vorliegt.

Der Reaktor ist in der Regel ein mit dem Ni-Katalysator beschickter zylindrischer Reaktor; alternativ kann eine Kaskade aus mehreren, vorzugsweise zwei bis drei, hintereinander geschalteten derartigen Reaktoren eingesetzt werden.

In dem Reaktor oder den einzelnen Reaktoren der Reaktorkaskade kann der Ni-Katalysator in einem einzigen oder in mehreren Ni-Katalysator-Festbetten angeordnet sein. Außerdem ist es möglich, in den einzelnen Reaktoren der Kaskade unterschiedliche Ni-Katalysatoren einzusetzen. Weiterhin können in den einzelnen Reaktoren der Reaktorkaskade unterschiedliche Reaktionsbedingungen hinsichtlich Druck und/oder Temperatur im Rahmen der obengenannten Druck- und Temperaturbereiche eingestellt werden.

Die vordere Reaktionsstufe sollte dabei bei > 50%, bevorzugt > 70% und besonders bevorzugt bei > 90% Gesamtolefinumsatz betrieben werden, während die hintere Reaktionsstufe den Restumsatz gewährleistet, so dass insgesamt ein Gesamtolefinumsatz von > 91 %, bevorzugt > 95% und besonders bevorzugt > 97% resultiert. Dies ist grundsätzlich auch unter Einsatz des Katalysators der vorderen Reaktionsstufe alleine möglich, erfordert aber im Vergleich zur Erfindung entweder hohe Reaktionstemperaturen, die zu einer relativ schnellen Katalysatordesaktivierung führen, oder große Katalysatorvolumina, die die Wirtschaftlichkeit des Verfahrens in Frage stellen würden.

Die vordere und die hintere Reaktionsstufe können dabei aus jeweils einem oder mehreren hintereinandergeschalteten Reaktoren bestehen, wie in WO 99/25668 und 01/72670 beschrieben.

Die erhaltenen 2-Buten-Oligomere können anschließend in die FCC-Anlage zurückgeführt werden, wobei durch Cracken und Isomerisierungsreaktionen unter anderem wiederum 1-Buten erzeugt wird. Insgesamt ergibt sich somit einer erhöhte Ausbeute an 1,3-Butadien.

Das ungenutzte n-Butan steht für den Verkauf zur Verfügung.

Typischerweise werden die in der Dehydrierstufe relativ inerten Butane im Stoffstrom f2 im Überschuss gegenüber den 2-Butenen vorliegen, wie es z.B. für eine nachgelagerte Alkylierung von Vorteil ist. Gegebenenfalls kann zusätzliches iso-Butan zugeführt werden, um ein bevorzugtes Verhältnis an Reaktionspartnern zu erhalten. Das zusätzliche zugeführte iso-Butan kann dabei auch durch Rückführung von nicht abreagiertem iso-Butan bereitgestellt werden.

In einem weiteren Schritt H) wird der 1,3-Butadien und das selektive Lösungsmittel enthaltenden Stoffstrom f1 destilliert und in einen das selektive Lösungsmittel enthaltenden Stoffstrom h1 und einen 1,3-Butadien enthaltenden Produktgasstrom h2 aufgetrennt.

Am Sumpf der Destillationskolonne fällt der Extraktionsmittel enthaltende Stoffstrom h1 an, wobei die Zusammensetzung des Extraktionsmittelstroms h1 im Wesentlichen der Zusammensetzung des Extraktionsmittels bei Zugabe zur der jeweiligen Extraktionsstufe entspricht. Der Extraktionsmittel enthaltende Stoffstrom h1 kann in die Extraktivdestillationsstufe F) zurückgeführt werden.

Im Allgemeinen wird ein Teil des Extraktionsmittelstroms h1 einer Extraktionsmittel-Aufreinigungsstufe zugeführt, im Allgemeinen 0,01 bis 0,5 % des Gesamtstroms des Lösemittelkreislaufs. Dazu kann das Lösemittel aus dem Sumpf einer Desorber-Kolonne kontinuierlich oder diskontinuierlich abgezogen und kontinuierlich oder diskontinuierlich abdestilliert werden. Bevorzugt erfolgt die Destillation diskontinuierlich. Nach Kondensation des Lösemittels kann das Lösemittel in die Extraktivdestillationen zurückgeführt werden.

1,3-Butadien kann über Kopf oder im Seitenstrom in einer Desorptionskolonne gewonnen werden. Falls das 1,3-Butadien über einen Seitenabzug (wie beispielsweise im Kapitel Butadiene in Ullmann's Encyclopedia of Industrial Chemistry 2012 in Fig. 3 für das sogenannte BASF-Verfahren beschrieben) gewonnen wird, wird die so abgezogene Extraktionslösung in eine Desorptionszone überführt, wobei aus der Extraktionslösung das 1,3-Butadien nochmals desorbiert und rückgewaschen wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% 1,3-Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

In dem 1,3-Butadien enthaltenden Stoffstrom h2 können noch weitere Verunreinigungen enthalten sein, beispielsweise Propin (Methylacetylen), 1,2-Butadien, C₅-Kohlenwasserstoffe oder ggf. auch geringe Mengen von Oxygenaten. In einer Ausführungsform des Verfahrens werden in einem oder mehreren weiteren Schritten aus dem Stoffstrom h2 Hochsieder entfernt. Dies erfolgt beispielsweise durch eine zweistufige Destillation. In einer ersten Destillation können Hochsieder wie beispielsweise Propin über Kopf abgetrennt werden. In einer zweiten Destillation des Sumpfstroms der vorangegangenen Kolonne kann 1,3-Butadie über Kopf abgetrennt werden, währen Schwersieder wie beispielsweise 1,2-Butadien oder C₅-Kohlenwasserstoffe im Sumpfstrom verbleiben. Das Spektrum der Verunreinigungen im Stoffstrom h2 hängt von verschiedenen Parametern ab. C₅-Kohlenwasserstoffe, 1,2-Butadien oder Propin werden im Allgemeinen über den Stoffstrom b1 in den Stoffstrom h2 gelangen. Das Spektrum der Verunreinigungen im Stoffstrom b1 hängt auch von der Zusammensetzung des eingesetzten Cracker-Feeds und der Crack-Schärfe ab. Oxygenate werden gegebenenfalls aus der Dehydrierstufe in den Stoffstrom f1 geschleppt und können über diesen gegebenenfalls bis in den Stoffstrom h2 gelangen. Das Spektrum der Verunreinigungen im Stoffstrom f1 hängt auch von den Verfahrensbedingungen in der Dehydrierstufe und der Trennleistung in den Verfahrensstufen D, Ea) und Eb) ab. Die Verfahrensstufe E) wird im Allgemeinen so ausgelegt sein, dass gegebenenfalls noch vorhandene Oxygenate im Stoffstrom e1 im Wesentlichen in den Stoffstrom f1 abgetrennt werden, um in den Stoffstrom f2 zu gelangen.

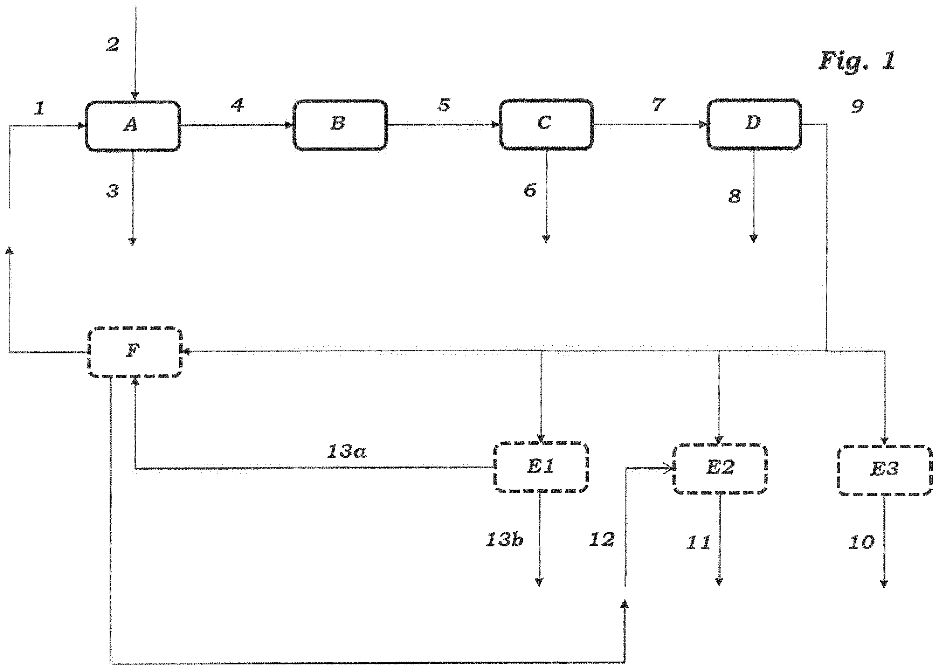

Figur 1 zeigt schematisch eine Ausführungsform des erfindungsgemäßen Verfahrens. Darin bedeuten:
- A: Stufe zur Abtrennung von iso-Buten
- B: Dehydrierstufe
- C: Absorptions- und Desorptionsstufe
- D: Extraktivdestillationsstufe
- E1: Einheit zur Oligomerisierung
- E2: Einheit zur Metathese und Bildung von Propylen
- E3: Alkylierung
- F: Riser einer FCC-Anlage, optional der FCC-Anlage die zur Erzeugung des Einsatzstroms 1 dient
- 1: C4-Produktfraktion aus einer FCC-Anlage enthaltend 1,3-Butadien, 1-Buten, cis- und trans-2-Buten, iso-Buten, iso-Butan, n-Butan
- 2: Methanol oder Ethanol
- 3: Strom enthaltend iso-Buten-Derivat, beispielsweise Methyl-tert.-butylether oder Ethyl-tert.-butylether
- 4: Strom enthaltend 1-Buten, 2-Butene, iso-Butan, n-Butan
- 5: Produktgasstrom der Dehydrierung
- 6: nicht kondensierbare Bestandteile
- 7: Strom enthaltend 1,3-Butadien, 1-Buten, 2-Butene, iso-Butan, n-Butan
- 8: Extraktionslösemittelstrom enthaltend 1,3-Butadien
- 9: Strom enthaltend 2-Butene, iso-Butan, n-Butan und ggfs. 1-Buten
- 10: Strom enthaltend Alkylate, n-Butan
- 11: Strom enthaltend Propylen
- 12: Ethylen (optional aus der FCC-Anlage die zur Erzeugung des Einsatzstroms 1 dient)
- 13: Strom enthaltend Oligomere, bevorzugt C₈ und C₁₂-Oligomere

## Patentansprüche

1. Verfahren zur Herstellung von 1,3-Butadien aus n-Butenen mit den Schritten:
A) Bereitstellung eines Butane, 1-Buten, 2-Buten, iso-Buten und gegebenenfalls 1,3-Butadien enthaltenden Einsatzgasstromes a aus einer Fluid-Catalytic-Cracking-Anlage;
B) Abtrennung von iso-Buten aus dem Einsatzgasstrom a, wobei ein Butane, 1-Buten, 2-Buten und gegebenenfalls 1,3-Butadien enthaltender Stoffstrom b erhalten wird;
C) Einspeisung des Butane, 1-Buten und 2-Buten enthaltenden Stoffstroms b und gegebenenfalls eines sauerstoffhaltigen Gases sowie gegebenenfalls Wasserdampf in mindestens eine Dehydrierzone und Dehydrierung von 1-Buten und 2-Buten zu 1,3-Butadien, wobei ein Produktgasstrom c enthaltend 1,3-Butadien, Butane, 2-Buten, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird;
D) Abkühlung und Kompression des Produktgasstroms c, wobei mindestens ein wässriger Kondensatstrom d1 und ein Gasstrom d2 enthaltend 1,3-Butadien, Butane, 2-Buten, Wasserdampf, gegebenenfalls Sauerstoff, leicht siedende Kohlenwasserstoffe, gegebenenfalls Kohlenstoffoxide und gegebenenfalls Inertgase erhalten wird;
Ea) Abtrennung von nicht kondensierbaren und leicht siedenden Gasbestandteilen umfassend leicht siedende Kohlenwasserstoffe, gegebenenfalls Sauerstoff, gegebenenfalls Kohlenstoffoxide und gegebenenfalls Inertgase als Gasstrom e2 aus dem Gasstrom d2 durch Absorption der C₄-Kohlenwasserstoffe umfassend 1,3-Butadien, Butane und 2-Buten in einem Absorptionsmittel, wobei ein mit C₄-Kohlenwasserstoffen beladener Absorptionsmittelstrom und der Gasstrom e2 erhalten werden, und
Eb) anschließende Desorption der C₄-Kohlenwasserstoffe aus dem beladenen Absorptionsmittelstrom, wobei ein C₄-Kohlenwasserstoffstrom e1 erhalten wird;
F) Auftrennung des C₄-Kohlenwasserstoffstroms e1 durch Extraktivdestillation mit einem für 1,3-Butadien selektiven Lösungsmittel in einen 1,3-Butadien und das selektive Lösungsmittel enthaltenden Stoffstrom f1 und einen Butane und 2-Buten enthaltenden Stoffstrom f2,
**dadurch gekennzeichnet, dass**
in Schritt C) mindestens 90% des in Strom b enthaltenen 1-Buten umgesetzt werden und in Schritt F ein Butane und 2-Buten enthaltender Produktstrom f2 erhalten wird.

2. Verfahren nach Anspruch 1, **dadurch gekennzeichnet, dass** der in Schritt F) erhaltene, Butane und 2-Buten enthaltende Stoffstrom f2 weiter umgesetzt wird gemäß einem oder mehreren der Schritte G1), G2) oder G3) durch
G1) Alkylierung von 2-Buten mit Butanen zu Isooctanen;
G2) Olefin-Metathese von 2-Buten mit Ethen zu Propen;
G3) Oligomerisierung von 2-Buten.

3. Verfahren nach Anspruch 1 oder 2, **dadurch gekennzeichnet, dass** die Dehydrierung in Schritt C) als oxidative Dehydrierung durchgeführt wird.

4. Verfahren nach einem der Ansprüche 1 bis 3, **dadurch gekennzeichnet, dass** in Schritt G3) C₈- und C₁₂-Oligomere des 2-Butens hergestellt werden.

5. Verfahren nach einem der Ansprüche 1 bis 4, **dadurch gekennzeichnet, dass** die in Schritt G3) erhaltenen 2-Buten-Oligomere in einer Fluid-Catalytic-Cracking-Anlage eingespeist werden.

6. Verfahren nach einem der Ansprüche 1 bis 5, **dadurch gekennzeichnet, dass** Schritt D) die Schritte umfasst:
Da) Abkühlung des Produktgasstroms d durch Inkontaktbringen mit einem Kühlmittel und Kondensation zumindest eines Teils der hochsiedenden Nebenkomponenten;
Db) Kompression des verbleibenden Produktgasstroms d in mindestens einer Kompressionsstufe, wobei mindestens ein wässriger Kondensatstrom e1 und ein Gasstrom e2 enthaltend 1,3-Butadien, Butane, n-Butene, Wasserdampf, gegebenenfalls Sauerstoff, leicht siedende Kohlenwasserstoffe, gegebenenfalls Kohlenstoffoxide und gegebenenfalls Inertgase erhalten wird.

7. Verfahren nach einem der Ansprüche 1 bis 6, **dadurch gekennzeichnet, dass** das in Schritt Ea) eingesetzte Absorptionsmittel ein aromatisches Kohlenwasserstofflösungsmittel ist.

8. Verfahren nach einem der Ansprüche 1 bis 7, **dadurch gekennzeichnet, dass** das in Schritten F) eingesetzte, für 1,3-Butadien selektive Lösungsmittel N-Methylpyrrolidon enthält.

9. Verfahren nach einem der Ansprüche 1 bis 8 mit dem Schritt
H) Destillation des 1,3-Butadien und das selektive Lösungsmittel enthaltenden Stoffstroms f1 aus der Stufe F) und Auftrennung in einen das selektive Lösungsmittel enthaltenden Stoffstrom h1 und einen 1,3-Butadien enthaltenden Produktgasstrom h2.

## Claims

1. A process for preparing 1,3-butadiene from n-butenes, comprising the steps of:
A) providing an input gas stream a comprising butanes, 1-butene, 2-butene and isobutene, with or without 1,3-butadiene, from a fluid catalytic cracking plant;
B) removing isobutene from the input gas stream a, giving a stream b comprising butanes, 1-butene and 2-butene, with or without 1,3-butadiene;
C) feeding the stream b comprising butanes, 1-butene and 2-butene and optionally an oxygenous gas and optionally water vapor into at least one dehydrogenating zone and dehydrogenating 1-butene and 2-butene to 1,3-butadiene, giving a product gas stream c comprising 1,3-butadiene, butanes, 2-butene and water vapor, with or without oxygen, with low-boiling hydrocarbons, with high-boiling secondary components, with or without carbon oxides and with or without inert gases;
D) cooling and compressing the product gas stream c, giving at least one aqueous condensate stream d1 and a gas stream d2 comprising 1,3-butadiene, butanes, 2-butene and water vapor, with or without oxygen, with low-boiling hydrocarbons, with or without carbon oxides and with or without inert gases;
Ea) removing uncondensable and low-boiling gas constituents comprising low-boiling hydrocarbons, with or without oxygen, with or without carbon oxides and with or without inert gases, as gas stream e2 from the gas stream d2 by absorbing the C4 hydrocarbons comprising 1,3-butadiene, butanes and 2-butene in an absorbent, giving an absorbent stream laden with C4 hydrocarbons and the gas stream e2, and
Eb) subsequently desorbing the C4 hydrocarbons from the laden absorbent stream, giving a C4 hydrocarbon stream e1;
F) separating the C4 hydrocarbon stream e1 by extractive distillation with a 1,3-butadiene-selective solvent into a stream f1 comprising 1,3-butadiene and the selective solvent and a stream f2 comprising butanes and 2-butene,
wherein
at least 90% of the 1-butene present in stream b is converted in step C) and a product stream f2 comprising butanes and 2-butene is obtained in step F.

2. The process according to claim 1, wherein the stream f2 which comprises butanes and 2-butene and is obtained in step F) is converted further in one or more of steps G1), G2) or G3) by
G1) alkylating 2-butene with butanes to give isooctanes;
G2) subjecting 2-butene to olefin metathesis with ethene to give propene;
G3) oligomerizing 2-butene.

3. The process according to claim 1 or 2, wherein the dehydrogenation in step C) is performed as an oxidative dehydrogenation.

4. The process according to any of claims 1 to 3, wherein C8 and C12 oligomers of 2-butene are prepared in step G3).

5. The process according to any of claims 1 to 4, wherein the 2-butene oligomers obtained in step G3) are fed into a fluid catalytic cracking plant.

6. The process according to any of claims 1 to 5, wherein step D) comprises the steps of:
Da) cooling the product gas stream d by contacting it with a coolant and condensing at least a portion of the high-boiling secondary components;
Db) compressing the remaining product gas stream d in at least one compression stage, giving at least one aqueous condensate stream e1 and one gas stream e2 comprising 1,3-butadiene, butanes, n-butenes and water vapor, with or without oxygen, with low-boiling hydrocarbons, with or without carbon oxides and with or without inert gases.

7. The process according to any of claims 1 to 6, wherein the absorbent used in step Ea) is an aromatic hydrocarbon solvent.

8. The process according to any of claims 1 to 7, wherein the 1,3-butadiene-selective solvent used in steps F) comprises N-methylpyrrolidone.

9. The process according to any of claims 1 to 8, comprising the step of:
H) distilling the stream f1 comprising 1,3-butadiene and the selective solvent from stage F) and separating it into a stream h1 comprising the selective solvent and a product gas stream h2 comprising 1,3-butadiene.

## Revendications

1. Procédé pour la préparation de 1,3-butadiène à partir de n-butènes présentant les étapes :
A) préparation d'un flux gazeux de départ a contenant des butanes, du 1-butène, du 2-butène, de l'isobutène et le cas échéant du 1,3-butadiène à partir d'une installation de craquage catalytique fluide ;
B) séparation de l'isobutène du flux gazeux de départ a, un flux de substances b contenant des butanes, du 1-butène, du 2-butène et le cas échéant du 1,3-butadiène étant obtenu ;
C) injection du flux de substances b contenant des butanes, du 1-butène et du 2-butène et le cas échéant d'un gaz contenant de l'oxygène ainsi que le cas échéant de vapeur d'eau dans au moins une zone de déshydrogénation et déshydrogénation du 1-butène et du 2-butène en 1,3-butadiène, un flux gazeux produit c contenant du 1,3-butadiène, des butanes, du 2-butène, de la vapeur d'eau, le cas échéant de l'oxygène, des hydrocarbures à bas point d'ébullition, des composants secondaires à point d'ébullition élevé, le cas échéant des oxydes de carbone et le cas échéant des gaz inertes étant obtenu ;
D) refroidissement et compression du flux gazeux produit c, au moins un flux de condensat aqueux d1 et un flux gazeux d2, contenant du 1,3-butadiène, des butanes, du 2-butène, de la vapeur d'eau, le cas échéant de l'oxygène, des hydrocarbures à bas point d'ébullition, le cas échéant des oxydes de carbone et le cas échéant des gaz inertes étant obtenus ;
Ea) séparation, à partir du flux gazeux d2, des constituants gazeux non condensables et de bas point d'ébullition, comprenant des hydrocarbures à bas point d'ébullition, le cas échéant de l'oxygène, le cas échéant des oxydes de carbone et le cas échéant des gaz inertes, sous forme de flux gazeux e2, par absorption des hydrocarbures en C₄ comprenant du 1,3-butadiène, des butanes et du 2-butène dans un agent d'absorption, un flux d'agent d'absorption chargé d'hydrocarbures en C₄ et le flux gazeux e2 étant obtenus et
Eb) désorption consécutive des hydrocarbures en C₄ du flux d'agent d'absorption chargé, un flux d'hydrocarbures en C₄ e1 étant obtenu ;
F) séparation du flux d'hydrocarbures en C₄ e1 par distillation extractive à l'aide d'un solvant sélectif pour le 1,3-butadiène en un flux de substances f1 contenant du 1,3-butadiène et le solvant sélectif et en un flux de substances f2 contenant des butanes et du 2-butène,
**caractérisé en ce que**,
dans l'étape C), au moins 90% du 1-butène contenu dans le flux b sont transformés et, dans l'étape F), un flux produit f2 contenant des butanes et du 2-butène est obtenu.

2. Procédé selon la revendication 1, **caractérisé en ce que** le flux de substances f2, obtenu dans l'étape F), contenant des butanes et du 2-butène, est transformé davantage selon l'une ou plusieurs des étapes G1), G2) ou G3) par
G1) alkylation de 2-butène avec des butanes en isooctanes ;
G2) métathèse d'oléfines de 2-butène avec de l'éthylène en propène ;
G3) oligomérisation de 2-butène.

3. Procédé selon la revendication 1 ou 2, **caractérisé en ce que** la déshydrogénation dans l'étape C) est réalisée sous forme de déshydrogénation oxydante.

4. Procédé selon l'une quelconque des revendications 1 à 3, **caractérisé en ce que**, dans l'étape G3), des oligomères en C₈ et en C₁₂ du 2-butène sont préparés.

5. Procédé selon l'une quelconque des revendications 1 à 4, **caractérisé en ce que** les oligomères du 2-butène, obtenus dans l'étape G3), sont injectés dans une installation de craquage catalytique fluide.

6. Procédé selon l'une quelconque des revendications 1 à 5, **caractérisé en ce que** l'étape D) comprend les étapes :
Da) refroidissement du flux gazeux produit d par mise en contact avec un agent de refroidissement et condensation d'au moins une partie des composants secondaires à point d'ébullition élevée ;
Db) compression du flux gazeux produit d qui reste dans au moins une étape de compression, au moins un flux de condensat aqueux e1 et un flux gazeux e2, contenant du 1,3-butadiène, des butanes, des n-butènes, de la vapeur d'eau, le cas échéant de l'oxygène, des hydrocarbures à bas point d'ébullition, le cas échéant des oxydes de carbone et le cas échéant des gaz inertes étant obtenus.

7. Procédé selon l'une quelconque des revendications 1 à 6, **caractérisé en ce que** l'agent d'absorption utilisé dans l'étape Ea) est un solvant hydrocarboné aromatique.

8. Procédé selon l'une quelconque des revendications 1 à 7, **caractérisé en ce que** le solvant sélectif pour le 1,3-butadiène, utilisé dans les étapes F) contient de la N-méthylpyrrolidone.

9. Procédé selon l'une quelconque des revendications 1 à 8, présentant l'étape
H) distillation du flux de substances f1, contenant du 1,3-butadiène et le solvant sélectif, provenant de l'étape F) et séparation en un flux de substances h1 contenant le solvant sélectif et en un flux gazeux produit h2 contenant du 1,3-butadiène.
